# EUROPEAN PATENT SPECIFICATION

(11) **EP 1 011 646 B1**
(45) Date of publication and mention of the grant of the patent: **17.08.2005**
(21) Application number: 97947786.6
(22) Date of filing: 04.12.1997
(51) Int. Cl.: A61K 9/72, B65D 83/14

(54) **PHARMACEUTICAL COMPOSITIONS AND DEVICES FOR THEIR ADMINISTRATION**
ARZNEIMITTEL ZUSAMMENSETZUNGEN UND VORRICHTUNGEN ZU DEREN VERABREICHUNG
COMPOSITIONS PHARMACEUTIQUES ET LEURS DISPOSITIFS D'ADMINISTRATION

(30) Priority: 04.12.1996 GB 9625171; 20.12.1996 GB 9626449
(43) Date of publication of application: 28.06.2000
(73) Proprietor: Link Products Limited, Horsham, West sussex RH10 1AH (GB)
(72) Inventor: McCARTHY, Paul, Clonmel, County Tipperary (IE); GOODMAN, Michael, Ampthill, Bedfordshire MK45 2XE (GB); LINDAHL, Ake, S-274 33 Skurup (SE)
(74) Representative: Jump, Timothy John Simon
(86) International application number: PCT/GB1997/003360
(87) International publication number: WO 1998/024420

(56) References cited:
- EP-A- 0 234 500
- EP-A- 0 518 601
- EP-A- 0 626 173
- WO-A-92/06675
- WO-A-96/32345
- WO-A-97/09034

## Description

The present invention relates to pharmaceutical compositions comprising a pharmaceutically active agent in liquified 1.1.1.2-tetrafluoroethane (HFC-134a) or 1,1,1,2,3,3,3-heptafluoropropane (HFC-227) as a propellant, for delivery in aerosol form, and to a device for delivering such a composition as an aerosol.

Most current aerosol spray formulations use one or more chlorofluorocarbon as a propellant; dichloro-difluoromethane being commonly used. However, chlorofluorocarbons have been implicated in the depletion of the ozone layer and their production, therefore, is being phased out. It has been found that certain hydrofluorocarbons, which are both of low toxicity and of suitable vapour pressure for use as aerosol propellants, are significantly less harmful to the ozone layer. Among such hydrofluorocarbons, 1,1,1,2-tetrafluoroethane (HFC-134a) and 1,1,1,2,3,3,3-heptafluoropropane (HFC-227) have been proposed as suitable propellants for pharmaceutical aerosols.

It has now been found that HFC-134a and HFC-227 can be used in combination with many pharmaceutically active agents, without causing any degradation to them or reducing their physiological activity.

Devices for administering metered aerosol doses of pharmaceutical preparations are well known in the art. Such devices include those disclosed in WO 92/11190, US-A-4819834 and US-A-4407481. Many of these devices include metering valves having components formed from plastic materials, such as the valves available from Bespak PLC of Bergen Way, Kings Lynn, Norfolk PE30 2JJ, United Kingdom, in which the valve core, metering chamber and some other structural components are formed from plastic materials. The plastic materials currently used for forming these structural parts in valves employed with many chlorofluorocarbon containing formulations include certain acetal co-polymers.

WO96/32345 discloses a metered dose inhaler device, for use with beclomethasone diproprionate in a formulation comprising a flurocarbon propellant, wherein all or part of the internal metallic surfaces of the device are coated with one or more fluorcarbon polymers.

WO97/09034 discloses an aerosol device for use with glyceryl trinitrate in a formulation comprising HFC-134a or HFC-227 as a propellant, wherein at least a portion of the device is formed from a polyester.

Although the plastics employed to manufacture metering valves, including the aforementioned acetal co-polymers, have also been found to be stable in the presence of HFC-134a alone, the applicants, to their surprise, have determined that many of these plastics materials can be caused to swell in the presence of formulations which include certain carriers or active agent solubilising co-solvents with HFC-134a. When such swelling takes place in a valve, the fit of mutually slidable components, such as metering chambers and valve cores, is adversely effected and they can bind together or become loose, causing the valve to leak or cease functioning altogether.

This problem has now been solved in accordance with a first aspect of the invention by a device for providing pharmaceutical doses comprising a container, filled with a pharmaceutical composition including a pharmaceutically active agent in a solution of liquified HFC-134a, or HFC-227, and a carrier selected from pharmaceutically acceptable alcohols, polyols, (poly) alkoxy derivatives, fatty acid alkyl esters, polyalkylene glycols, and dimethylsulphoxide, and valve means arranged for delivering aerosol doses of said pharmaceutical composition to the exterior of the container, wherein at least a portion of the device is formed from a polyester and the pharmaceutical composition includes no glyceryl trinitrate.

Preferably, the valve means includes at least one component formed from a polyester, which component, more preferably, is a metering chamber and/or a valve core. Preferably, devices in accordance with the invention are arranged to provide metered doses of the pharmaceutically active agent included therein.

In further embodiments, the container comprises a polyester and, preferably, consists of metal lined with a polyester. The canister cap can also be so formed.

Apart from allowing the aforementioned swelling problem to be solved, an advantage of this aspect of the present invention is that use of expensive metal valve components can be avoided.

During the course of the work leading to this aspect of the present invention, tests carried out on active agent/carrier or co-solvent/HFC-134a filled metered dose aerosol devices, with acetyl copolymer or nylon valve components, showed that they failed to provide uniform doses after storage under controlled conditions. Such effects are normally associated with problems involving the gaskets or seals employed within the valve mechanisms. Thus, it came as a surprise to the applicants when they discovered that these failures were being caused by the valve components swelling to an unacceptable extent, particularly since at least one of the materials used to form them (acetyl co-polymer) was known to be stable in the presence of HFC-134a alone, or conventional active agent/carrier or co-solvent/chlorofluorocarbon formulations.

The preferred polyesters are polyalkylene benzene dicarboxylates, more preferably polyalkylene terephthalates and, most preferably, a polybutylene terephthalate.

Such materials, preferably, have a density of about 1.3g/cm³ and a water absorption of about 0.6% (23°C saturation). The polyesters, also, are preferably partially crystalline in nature and have a crystalline melting range of 220-225°C.

Examples of suitable polybutylene terephthalates include those available under the Trademark Celanex® from Hoechst UK Limited, Walton Manner, Milton Keynes, Bucks MK7 7AJ, United Kingdom. Particularly preferred are Celanex® 2500 and Celanex® X 500/2.

Preferably, the carrier is a lower alkyl (C₁-C₄) alcohol, a polyol, or a (poly) alkoxy derivative. In embodiments, the carrier is a C₁-C₄ alkyl alcohol or a lanolin alcohol and, preferably, is ethanol or isopropyl alcohol. The most preferred alcohol is ethanol.

The preferred polyols include propylene glycol and glycerol and the preferred (poly) alkoxy derivatives include polyalkoxy alcohols, in particular 2-(2-ethoxyethoxy) ethanol (available under the Trademark Transcutol®).

Further preferred (poly)alkoxy derivatives include polyoxyalkyl ethers and esters, such as polyoxyethylene ethers or esters. The preferred polyoxyethylene ethers and esters are polyoxyethylene alkyl ethers, polyoxyethylene castor oil derivatives, polyoxyethylene sorbitan fatty acid esters and polyoxyethylene stearates.

The preferred fatty acid alkyl esters are ethyl oleate, isopropyl myristate and isopropyl palmitate. The preferred polyalkylene glycol is polyethylene glycol.

In preferred embodiments, the inventive composition can comprise up to 50% or, preferably, 25% w/w carrier. More preferred embodiments include between 3% and 15% w/w, or between 4 and 10% w/w carrier. The pharmaceutical compositions can comprise between 50% and 99% w/w, preferably between 75% and 99% w/w, and, more preferably, between 88% and 95% w/w HFC-134a or HFC-227.

In further embodiments, compositions used in the present invention can comprise a plurality of different carriers.

Further excipients can be included in the formulations employed in the present invention. For example, neutral oils as well as surfactants (the latter for aiding the smooth operation of the valve), as are well known to those skilled in the art, may be included.

Thus, in further preferred embodiments, compositions employed in the invention can comprise an organic surfactant. The preferred organic surfactant is oleyl alcohol, although others can be employed, including sorbitan trioleate, sorbitan mono-oleate, sorbitan monolaurate; polyoxyethylene (20) sorbitan monolaurate, polyoxyethylene (20) sorbitan mono-oleate, natural lecithin, oleyl polyoxytheylene (2) ether, stearyl polyoxyethylene (2) ether, lauryl polyoxyethylene (4) ether, block copolymers of oxyethylene and oxypropylene, oleic acid, synthetic lecithin, diethylene glycol dioleate, tetrahydrofurfuryl oleate, ethyl oleate, isopropyl myristate, glyceryl mono-oleate, glyceryl monostearate, glyceryl monoricinoleate, cetyl alcohol, stearyl alcohol, cetyl pyridinium chloride, olive oil, glyceryl monolaurate, corn oil, cotton seed oil or sunflower seed oil.

The pharmaceutically active agent, preferably, is insoluble, or only sparingly soluble in pure liquified HFC-134a or HFC-227. Preferably, the solubility of the active agent in liquified HFC-134a or HFC-227 is from 3 to 0.001%w/v, preferably from 1 to 0.01% w/v. However, in certain preferred embodiments, the solubility of the active agent in liquified HFC-134a or HFC-227 is from 3 to 1% w/v.

The preferred active agents include:-
(1) steroid drugs such as, for example, beclomethasone, betamethasone, dexamethasone, fluticasone, hydrocortisone, budesonide, flunisolide, triamcinolone flumethasone, and prednisolone;
(2) antibiotic and antibacterial agents such as, for example, neomycin, mupirocin and chlorhexidine;
(3) systemically active drugs such as, for example, isosorbide dinitrate, isosorbide mononitrate, apomorphine and nicotine;
(4) antihistamines such as, for example, azelastine, chlorpheniramine, astemizole and terfenadine;
(5) anti-inflammatory agents such as, for example, piroxicam, nedocromil, cromoglycate, fasafungine and iodoxamide;
(6) anticholinergic agents such as, for example, ipratropium bromide and oxitropium bromide;
(7) anti-emetics such as, for example, domperidone, hyoscine, cinnarizine metoclopramide, cyclizine, dimenhydrinate and promethazine;
(8) hormonal drugs such as, for example, vasopressin or desmopressin;
(9) bronchodilators, such as salbutamol, fenoterol and salmeterol;
(10) sympathomimetic drugs, such as tramazoline and xylometazoline;
(11) Anti-fungal drugs such as miconazole;
(12) Local anaesthetics such as benzocaine and lignocaine; and
(13) pharmaceutically acceptable salts of any of the foregoing.

Of these, the most preferred pharmaceutically active agent is beclomethasone dipropionate. Beclomethasone dipropionate may be employed in an anhydrous, hydrated or solvated state but, preferably, is employed in an anhydrous state. The preferred propellant is HFC-134a.

Preferably, the pharmaceutical composition includes a solution of the pharmaceutically active agent in HFC-134a or HFC-227, with the carrier as a co-solvent. It is further preferred that the co-solvent solubilises the active agent, in the sense that its presence increases the solubility of the active agent in the composition and, thus, causes or allows all or a proportion of the active agent present in the composition to dissolve and/or remain in solution.

The pharmaceutical compositions can be partial solutions in which only a proportion of the pharmaceutically active agent present therein is dissolved in the propellant and co-solvent, with the remainder being in suspension or suspendible. The exact proportions of dissolved and suspended active agent will depend upon the active agent concerned, its concentration and the identity and quantity of the co-solvent(s) used. In preferred embodiments the compositions are in the form of liquid solutions when maintained under pressure in devices in accordance with the invention.

A particularly preferred embodiment of the invention comprises a device in accordance with the first aspect of the invention filled with a solution of beclomethasone, preferably beclomethaone dipropionate, in ethanol as a co-solvent and HFC-134a as a propellant.

Devices and formulations in accordance with the invention can be used to provide sprays suitable for nasal, or sublingual administration, or for inhalation. Preferably, the compositions of this invention are formulated for administration to the nasal passages or the sublingual mucosa and devices in accordance with the invention are arranged for providing a spray of the inventive composition to either of the latter locations.

In embodiments wherein the composition is intended for sublingual administration, it can further comprises a flavouring oil such as, for example peppermint oil Ph Eur.

In a second aspect, the invention provides the use of a polyester in contact with a composition of the type present in devices in accordance with the first aspect of the invention. Preferably the polyester is one of those described above, and the use takes place in a metered dose dispensing aerosol device.

An embodiment of the first aspect of the present invention will now be described, by way of example only, and with reference to the following drawing.

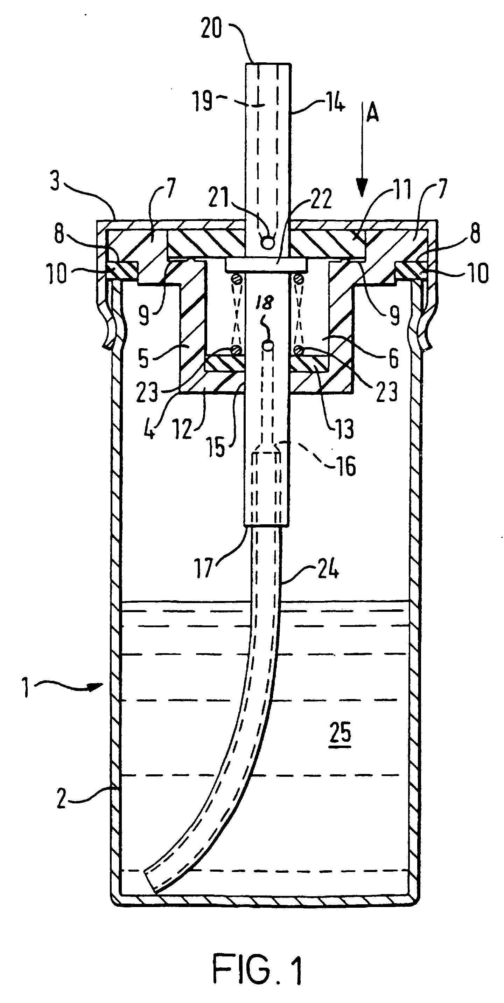

Figure 1 is a cross sectional view of an embodiment of a device in accordance with the invention.

The device 1 comprises a substantially cylindrical canister 2 sealed with a cap 3. Both the canister 2 and the cap 3 are formed from an aluminium alloy and can be lined with a polyester (such as Celanex® 2500) or a lacquer (not shown).

A valve body moulding 4 comprises a cylindrical portion 5, which defines a metering chamber 6 and a stepped flange portion 7, and is formed by injection moulding from Celanex® 2500. The stepped flange portion 7 defines a first and outwardly facing annular seat 8 and a second, inwardly facing annular seat 9. The first annular seat 8 accommodates an annular sealing ring 10 and the second annular seat 9 accommodates a first sealing washer 11. The first sealing washer 11 is located so as to cooperate with the cylindrical portion 5 of the valve body moulding 4, in defining the metering chamber 6.

A base 12 of the cylindrical portion 5 of the valve body moulding 4 completes the boundary to the metering chamber 6 and provides a seat for a second sealing washer 13.

The sealing ring 10 and the first and second sealing washers 11 and 13 can be formed from a butyl rubber, neoprene or one of the elastomers disclosed for such purposes in WO 92/11190.

An elongate, substantially cylindrical and partially hollow valve core 14 is slidably located within the first and second sealing washers 11 and 13 and extends through an orifice 15, defined in the base 12. The valve core 14 is formed by injection moulding from Celanex® 2500.

A stepped inlet passage 16 communicates between a first end 17 of the valve core 14 and an inlet orifice 18, formed through the side of the valve core 14. In a likewise manner, an outlet passage 19 communicates between the second end 20 of the valve core 14 and an outlet orifice 21 formed through the side of the valve core 14. An annular flange 22 extends radially outwardly from the valve core 14 between the inlet and outlet orifices 18 and 21 and adjacent to the outlet orifice 21.

A stainless steel compression coil spring 23 acts between the annular flange 22 and the second sealing washer 13, urging the annular flange 22 into contact with the first sealing washer 11, such that the outlet orifice 21 lies inside the first sealing washer 11 and is thereby isolated from the metering chamber 6. In this position, as shown in Figure 1, the inlet orifice 18 is located within the metering chamber 6. A flexible tube 24 is engaged within the stepped inlet passage 16 and extends from the valve core 14 to the base of the canister 2 (as shown in Figure 1). Thus, the inlet orifice 18 is in communication with a region within the canister 2 adjacent to its base 12.

The cap 3 is firmly attached to the canister 2 by crimping and, thus, holds the assembly of the valve body moulding 4, valve core 14, coil spring 23, sealing washers 11 and 13 and sealing ring 10 in place as shown in Figure 1, with the sealing ring 10 and first sealing washer 11 sufficiently compressed to seal the interior of the device 1 and prevent the egress of its contents.
Downward movement of the valve core, in the direction of arrow A, against the action of the spring 22 will bring the outlet orifice 21 into the metering chamber immediately after the first orifice 18 has been sealed from the metering chamber 6 by the second sealing washer 13.

When filled with a composition in accordance with the present invention, as shown at 25, the device 1 will provide metered doses of the composition when used as follows. The device 1 should be held in the position shown in Figure 1, so that the composition 25, by virtue of its pressure, enters the metering chamber 6 via the tube 24, the inlet passage 16 and the inlet orifice 18. Subsequent depression of the valve core 14, in the direction of arrow A, seals the inlet orifice 18 and hence the remainder of the canister 2, from the metering chamber 6 and opens the outlet passage to the metering chamber 6, via the outlet orifice 21. Since the composition 25 in the metering chamber 6 is pressurised with the propellant, it will be expelled from the metering chamber 6 through the outlet orifice 21 and the outlet passage 19. If the valve core 14 is then allowed to return to the position shown in Figure 1, under the influence of the spring 22, the outlet orifice 21 is again sealed from the metering chamber 6 and the metering chamber 6 will be filled with pressurised composition 25 from the canister 2, via the tube 24, stepped inlet passage 16 and inlet orifice 18.

### Example 1

A composition comprising beclomethasone dipropionate (BDP) with HFC-134a suitable for use in a device as described above was formulated from the following ingredients:-

| Component | percent w/w | g/can |
|---|---|---|
| BDP (anhydrous) | 0.164 | 0.010 |
| Ethanol 96% BP | 4.992 | 0.305 |
| HFC-134a | 94.844 | 5.795 |
| Total | 100 | 6.11 |

The BDP was dissolved in the ethanol in the proportions set out above and 0.315 g of the resulting solution was then placed in a canister 2 and a valve assembly, comprising a valve body moulding 4, first sealing washer 11, second sealing washer 13, spring 22, tube 23, and annular seal 10, was then sealed onto the canister 2 by crimping as shown in Figure 1 by the cap 3. The propellant (HFC-134a) was then added to the canister, by being forced through the valve core 14 at great pressure, and the complete device was then checked for leaks. After the propellant entered the canister it dissolved the remaining portions of the composition.

In a preferred embodiment, each expelled dose of the above formulation is of approximately 25µl and provides 50µg of BDP.

### Example 2

A second composition comprising BDP and suitable for use in a device as described above was formulated from the following ingredients:-

| Component | percent w/w | g/can |
|---|---|---|
| BDP (anhydrous) | 0.164 | 0.010 |
| Ethanol 96% BP | 7.5 | 0.458 |
| HFC-134a | 92.336 | 5.641 |
| Total | 100 | 6.11 |

The BDP was dissolved in the ethanol in the proportions set out above and 0.315g of the resulting solution was then placed in a canister 2. A valve assembly (as described in Example 1) was than sealed onto the canister 2 by crimping and the HFC-134a propellant was then added to the canister, by being forced through the valve core 14 at great pressure, and the complete device was then checked for leaks. After the propellant entered the canister it dissolved the remaining portions of the composition.

In a preferred embodiment, each expelled dose of the above formulation is approximately 25µl and provides 50µl of BDP.

### Example 3

Further compositions comprising BDP with HFC-134a, suitable for use in a device as described herein, were formulated in accordance with the details set out in the following table, in which all figures are given on a percent by weight basis.

| **Formulation** | **A** | **B** | **C** | **D** | **E** |
|---|---|---|---|---|---|
| BDP | 0.164 | 0.164 | 0.164 | 0.164 | 0.164 |
| Transcatol | 9.984 | 4.992 | | | |
| Oleyl alcohol | | | 2.496 | | |
| Propylene glycol | | | | 4.992 | |
| Ethanol | | 4.992 | 7.488 | 4.992 | 20.51 |
| p134a | 89.852 | 89.852 | 89.852 | 89.852 | 79.326 |
| Total | 100 | 100 | 100 | 100 | 100 |

Formulations A-E are prepared using a similar technique to that set out in example 1 above. Briefly, the BDP is dissolved with the other excipient or excipients (excepting the HFC-134a) and the resulting solution is then placed in a canister 2. A valve assembly is then sealed onto the canister 2 by crimping and the HFC-134a propellant is then added to the canister 2, by being forced through the valve core 14 at great pressure. After the propellant enters the canister 2, it dissolves the remaining portions of each composition.

Although only BDP is referred to in this example, other ones of the active agents previously discussed in this application may be substituted therefor in quantities which would dissolve at least partially in the propellant/co-solvent mixture.

### Examples 4-11

Eight further compositions suitable for use in a device as described herein, were formulated in accordance with the details set out in the following table.

| Example | Component | Percent w/w |
|---|---|---|
| Example 4 | Mupirocin | 0.1 |
| | Tween 20 | 0.1 |
| | Ethanol | 20.0 |
| | HFC-134a | 79.8 |
| | | |
| Example 5 | Isosorbide Dinitrate | 1.0 |
| | Propylene Glycol | 3.0 |
| | Peppermint Oil Ph Eur | 1.0 |
| | Ethanol | 15.0 |
| | HFC-134a | 80.0 |
| | | |
| Example 6 | Cromoglycate | 0.2 |
| | Span 5 | 0.1 |
| | Ethanol | 20.0 |
| | HFC-134a | 79.7 |
| | | |
| Example 7 | Domperidone | 0.2 |
| | Oleyl alcohol | 0.1 |
| | Transcutol | 4.0 |
| | Ethanol | 10.0 |
| | HFC-134a | 84.7 |
| | | |
| Example 8 | Salbutamol | 0.2 |
| | Oleic acid | 0.01 |
| | Miglyol 840 | 2.0 |
| | Ethanol | 10.0 |
| | HFC-134a | 87.89 |
| | | |
| Example 9 | Xylometazoline | 0.1 |
| | Oleic acid | 0.01 |
| | Propylene glycol | 3.0 |
| | Ethanol | 12.0 |
| | HFC-134a | 84.89 |
| | | |
| Example 10 | Miconazole | 1.0 |
| | Oleic acid | 0.1 |
| | Transcucol | 4.0 |
| | Ethanol | 16.0 |
| | HFC-134a | 78.9 |
| | | |
| Example 11 | Benzocaine | 1.0 |
| | PEG 400 | 4.0 |
| | Ethanol | 5.0 |
| | HFC-134a | 90.0 |

These compositions are prepared using a similar technique to that set out in Examples 1-2. Briefly, the active agent is mixed with the other excipient or excipients (excepting the HFC-134a) and the resulting solution and/or suspension is then placed in a canister 2. A valve assembly (as described in Example 1) is then sealed onto the canister 2 by crimping and the HFC-134a propellant is then added to the canister, by being forced through the valve core 14 at great pressure. After the propellant enters the canister 2, it at least partially and in some cases completely dissolves the remaining portions of each composition.

## Claims

1. A device for providing pharmaceutical doses comprising a container, filled with a pharmaceutical composition including a pharmaceutically active agent in a solution of liquified 1,1,1,2-tetrafluoroethane (HFC-134a), or 1,1,1,2,3,3,3-heptafluoropropane (HFC-227), and a carrier selected from pharmaceutically acceptable alcohols, polyols, (poly) alkoxy derivatives, fatty acid alkyl esters, polyalkylene glycols and dimethyl sulphoxide, and valve means arranged for delivering aerosol doses of said pharmaceutical composition to the exterior of the container, wherein at least a portion of the device is formed from a polyester and the pharmaceutical composition includes no glyceryl trinitrate.

2. A device as claimed in claim 1, wherein the carrier, is
(a) a C₁-C₄ lower alkyl alcohol or a lanolin alcohol;
(b) a polyalkoxy alcohol;
(c) a polyoxyalkyl ether or ester; and/or
(d) a polyoxyethylene alkyl ether, a polyoxyethylene caster oil derivative, a polyoxyethylene sorbitan fatty acid ester or a polyoxyethylene stearate.

3. A device as claimed in claim 2, wherein the polyoxyalkyl ether or ester is a polyoxyethylene ether or ester.

4. A device as claimed in claim 1, wherein the carrier is ethyl oleate, isopropyl myristate, isopropyl palmitate, polyethylene glycol, propylene glycol, glycerol, isopropyl alcohol, ethanol and/or 2-(2-ethoxy ethoxy) ethanol (Transcutol).

5. A device as claimed in any of the preceding claims wherein the composition comprises up to 50% w/w carrier and/or between 50 and 99% w/w HFC-134a or HFC-227.

6. A device as claimed in claim 5, wherein the composition comprises up to 25% w/w carrier.

7. A device as claimed in claim 5 or 6, wherein the composition comprises between 75 and 95% w/w HFC-134a or HFC-227.

8. A device as claimed in any of the preceding claims, wherein the composition further comprises an organic surfactant.

9. A device as claimed in claim 8, wherein the organic surfactant is oleyl alcohol, sorbitan trioleate, sorbitan mono-oleate, sorbitan monolaurate, polyoxyethylene (20) sorbitan monolaurate, polyoxyethylene (20) sorbitan mono-oleate, natural lecithin, oleyl polyoxyethylene (2) ether, stearyl polyoxyethylene (2) ether, lauryl polyoxyethylene (4) ether, block copolymers of oxyethylene and oxypropylene, oleic acid, synthetic lecithin, diethylene glycol dioleate, tetrahydrofurfuryl oleate, ethyl oleate, isopropyl myristate, glyceryl mono-oleate, glyceryl monostearate, glyceryl monoricinoleate, cetyl alcohol, stearyl alcohol, cetyl pyridinium chloride, olive oil, glyceryl monolaurate, corn oil, cotton seed oil or sunflower seed oil.

10. A device as claimed in claim 8, wherein the surfactant is oleyl alcohol.

11. A device as claimed in claim 1, wherein the pharmaceutically active agent is selected from:
(1) steroid drugs;
(2) antibiotic and antibacterial agents;
(3) systemically active drugs;
(4) antihistamines;
(5) anti-inflammatory agents;
(6) anticholinergic agents;
(7) anti-emetics;
(8) hormonal drugs;
(9) bronchodilators;
(10) sympathomimetic drugs;
(11) anti-fungal drugs;
(12) local anaesthetics; and
(13) pharmaceutically acceptable salts of any of the foregoing.

12. A device as claimed in claim 11, wherein:
(1) the steroid drugs are beclomethasone, betamethasone, dexamethasone, fluticasone, hydrocortisone, budesonide, flunisolide, triamcinolone flumethasone and prednisolone;
(2) the antibiotic or antibacterial agents are neomycin, mupirocin and chlorhexidine;
(3) the systemically active drugs are isosorbide dinitrate, isosorbide mononitrate, apomorphine and nicotine;
(4) the antihistamines are azelastine, chlorpheniramine, astemizole and terfenadine;
(5) the anti-inflammatory agents are piroxicam, nedocromil, cromoglycate, fasafungine and iodoxamide;
(6) the anticholinergic agents are ipratropium bromide and oxitropium bromide;
(7) the anti-emetics are domperidone, hyoscine, cinnarizine metoclopramide, cyclizine, dimenhydrinate and promethazine;
(8) the hormonal drugs are vasopressin and desmopressin;
(9) the bronchodilators are salbutamol, fenoterol and salmeterol;
(10) the sympathomimetic drugs are tramazoline and xylometazoline;
(11) the anti-fungal drug is miconazole; and
(12) the local anaesthetics are benzocaine and lignocaine.

13. A device as claimed in claim 11 or 12, wherein the active agent is beclomethasone dipropionate.

14. A device as claimed in claim 13, wherein the beclomethasone dipropionate is in an anhydrous, hydrated or solvated state.

15. A device as claimed in claim 14, wherein the beclomethasone dipropionate is in an anhydrous state.

16. A device as claimed in any of the preceding claims substantially free of weak organic or strong inorganic acid.

17. A device as claimed in any of the preceding claims, wherein the solubility of the active agent in liquified HFC-134a or HFC-227 is from 3-0.001% w/v.

18. A device as claimed in claim 17, wherein the solubility of the active agent in liquified HFC-134a or HFC-227 is from 1-0.01% w/v.

19. A device as claimed in any of the preceding claims, wherein the valve means includes at least one component formed from a polyester.

20. A device as claimed in claim 19, wherein two relatively moveable valve components are formed from a polyester.

21. A device as claimed in claim 20, wherein the two relatively moveable valve components are mutually slideable.

22. A device as claimed in claim 19, wherein said component is a metering chamber and/or is a valve core.

23. A device as claimed in any of the preceding claims, wherein the container includes a canister body comprising a polyester or a canister body formed from metal lined with the polyester.

24. A device as claimed in any of the preceding claims, wherein the polyester is a polyalkylene benzene dicarboxylate.

25. A device as claimed in claim 24, wherein the polyester is a polyalkylene terephthalate, optionally a polybutylene terephthalate.

26. A device as claimed in any of the preceding claims, wherein the composition further comprises a flavouring oil.

27. A device as claimed in claim 26, wherein the flavouring oil is peppermint oil.

28. A device as claimed in any of the preceding claims, wherein the pharmaceutical composition includes a solution of the pharmaceutically active agent in liquified HFC-134a or HFC-227, with the carrier as a co-solvent.

29. A device as claimed in any of claims 1-28, wherein the composition comprises HFC-134a, beclamethasone dipropionate and ethanol.

30. Use of a polyester in contact with a composition as defined in any of claims 1-19.

31. A use as claimed in claim 30, wherein the polyester is as defined in claim 24 or 25.

## Patentansprüche

1. Vorrichtung zur Bereitstellung von pharmazeutischen Dosen umfassend ein Gefäß, das mit einer pharmazeutischen Zusammensetzung gefüllt ist, die ein pharmazeutisch aktives Agens in einer Lösung aus verflüssigtem 1,1,1,2-Tetrafluorethan (HFC-134a) oder 1,1,1,2,3,3,3-Heplatluorpropan (HFC-227) und einen Träger umfasst, der ausgewählt ist aus pharmazeutisch akzeptablen Alkoholen, Polyolen, (Poly)Alkoxyderivaten, Fettsäurealkylestern, Polyalkylenglycolen und Dimethylsulfoxid, und ein Ventilmittel, das angeordnet ist, um Aerosoldosen der pharmazeutischen Zusammensetzung nach außerhalb des Gefäßes abzugeben, wobei wenigstens ein Teil der Vorrichtung aus einem Polyester hergestellt ist und die pharmazeutische Zusammensetzung kein Glyceroltrinitrat enthält.

2. Vorrichtung nach Anspruch 1, wobei der Träger
(a) ein niedriger C₁-C₄-Alkylalkohol oder ein Lanolin-Alkohol ist;
(b) ein Polyalkoxyalkohol ist;
(c) ein Polyoxyalkylether oder -ester ist; und/oder
(d) ein Polyoxyethylenalkylether, ein Polyoxyethylen-Rizinusölderivat, ein Polyoxyethylen-Sorbitan-Fettsäureester oder ein Polyoxyethylen-Stearat ist.

3. Vorrichtung nach Anspruch 2, wobei der Polyoxyalkylether oder -cster ein Polyoxyethylenether oder -ester ist.

4. Vorrichtung nach Anspruch 1, wobei der Träger Ethyloleat, Isopropylmyristat, Isopropylpalmitat, Polyethylenglycol, Propylenglycol, Glycerol, Isopropylalkohol, Ethanol und/oder 2-(2-Ethoxy-ethoxy) Ethanol (Transcutol) ist.

5. Vorrichtung nach einem der vorangehenden Ansprüche, wobei die Zusammensetzung bis zu 50 Gew./Gew.% Träger und/oder zwischen 50 und 99 Gew./Gcw.% HFC-134a oder HFC-227 umfasst.

6. Vorrichtung nach Anspruch 5, wobei die Zusammensetzung bis zu 25 Gew./Gew.% Träger umfasst.

7. Vorrichtung nach Anspruch 5 oder 6, wobei die Zusammensetzung zwischen 75 und 95 Gew./Gew.% HFC-134a oder HFC-227 umfasst.

8. Vorrichtung nach einem der vorangehenden Ansprüche, wobei die Zusammensetzung weiter ein organisches oberflächenaktives Mittel umfasst.

9. Vorrichtung nach Anspruch 8, wobei das organische oberflächenaktive Mittel Oleylalkohol, Sorbitantrioleat, Sorbitan-Monooleat, Sorbitan-Monolaurat, Polyoxyethylen (20)-Sorbitan-Monolaurat, Polyoxyethylen (20)-Sorbitan-Monooleat, natürliches Lccithin, Oleyl-Polyoxyethylen (2)-Ether, Stearyl-Polyoxyethylen (2)-Ether, Lauryl-Polyoxyethylen (4)-Ether, Blockcapolymere aus Oxyethylen und Oxypropylen, Olsäurt, synthetisches Lecithin, Diethylenglycoldiolcat, Tetrahydrofurfuryloleat, Ethyloleat, Isopropylmyristat, Glycerolmonooleat, Glycerolmonostearat, Glycerolmonoricinoleat, Cetylalkohol, Stearylalkohol, Cetylpyridiniumchlorid, Olivenöl, Glycerolmonolaurat, Maisöl, Baumwollsamenöl oder Sonnenblumenöl ist.

10. Vorrichtung nach Anspruch 8, wobei das oberflächenaktive Mittel Oleylalkohol ist.

11. Vorrichtung nach Anspruch 1, wobei das pharmazeutisch aktive Agens ausgewählt ist aus:
(1) Steroid-Arzneimittel;
(2) antibiotische und antibakterielle Agenzien;
(3) systemmisch aktive Arzneimittel;
(4) Antihistamine;
(5) anti-entzündliche Agenzien;
(6) Anticholinergika;
(7) Antiemetika;
(8) Hormon-Arzneimittel;
(9) Bronchodilatoren;
(10) Sympathomimetika;
(11) pilztötende Arzneimittel;
(12) Lokalanästhetika; und
(13) pharmazeutische akzeptable Salze von einem jeden der vorhergehenden Mittel.

12. Vorrichtung nach Anspruch 11, wobei:
(1) die Steroid-Arzneimittel Beclomethason, Betamethason, Dexamethason, Fluticason, Hydrocortison, Budesonid, Flunisolid, Triamcinolon, Flumethason und Prednisolon sind;
(2) die antibiotischen oder antibakteriellen Mittel Neomycin, Mupirocin und Cblorhexidin sind;
(3) die systcmisch aktiven Arzneimittel Isosorbid-Dinitrat, Tsosorbid-Mononitrat, Apomorphin und Nicotin sind;
(4) die Antihistamine Azelastin, Chlorpheniramin, Astemizol und Terfenadin sind;
(5) die anti-entzündlichen Agenzien Piroxicam, Nedocromil, Cromoglycat, Fasafungin und Indoxamid sind;
(6) die Anticholinergika Ipratropium-Bromid und Oxitropium-Bromid sind;
(7) die Antiemetika Domperidon, Hyoscin, Cinnarizin, Mctoclopramid, Cyclizin, Dimenhydrinat und Promethazin sind;
(8) die Hormon-Arzneimittel Vasopressin und Desmopressin sind;
(9) die Bronchodilateren Salbutamol, Fenoterol und Salmeterol sind;
(10) die Sympathomimetika Tramazolin und Xylometazolin sind;
(11) das pilztötende Arzneimittel Miconazol ist; und
(12) die Lokalanästhetika Benzocain und Lignocain sind.

13. Vorrichtung nach Anspruch 11 oder 12, wobei das aktive Agens Beclomethason-Dipropionat ist.

14. Vorrichtung nach Anspruch 13, wobei das Beclomethason-Dipropionat sich in einem wasserfreien, hydratisierten oder solvatisierten Zustand befindet.

15. Vorrichtung nach Anspruch 14, wobei das Beclomethason-Dipropionat sich in einem wasserfreien Zustand befindet.

16. Vorrichtung nach einem der vorhergehenden Ansprüche die im wesentlichen frei von einer schwachen organischen oder starken anorganischen Säure ist.

17. Vorrichtung nach einem der vorangehenden Ansprüche, wobei die Löslichkeit des aktiven Agens in verflüssigtem HFC-134a oder HFC-227 von 3-0,001 Gew./Vol.% ist.

18. Vorrichtung nach Anspruch 17, wobei die Löslichkeit des aktiven Agens in verflüssigtem HFC-134a oder HFC-227 von 1-0,01 Gew./Vol.% ist.

19. Vorrichtung nach einem der vorhergehenden Ansprüche, wobei das Ventilmittcl wenigstens einen Bestandteil umfasst, der aus einem Polyester hergestellt ist.

20. Vorrichtung nach Anspruch 19, wobei zwei relativ zueinander bewegliche Ventilbestandteile aus einem Polyester hergestellt sind.

21. Vorrichtung nach Anspruch 20, wobei zwei relativ zueinander bewegliche Ventilbestandteile wechselseitig verschiebbar sind.

22. Vorrichtung nach Anspruch 19, wobei der Bestandteil eine Dosier- und/oder ein Ventileinsatz ist.

23. Vorrichtung nach einem der vorangehenden Ansprüche, wobei das Gefäß einen Kanisterkörper, der einen Polyester umfasst, oder einen Kanisterkörper enthält, der aus Metall hergestellt ist, das mit dem Polyester ausgekleidet ist.

24. Vorrichtung nach einem der vorangehenden Ansprüche, wobei der Polyester ein Polyalkylenbenzendicarboxylat ist.

25. Vorrichtung nach Anspruch 24, wobei der Polyester ein Polyalkylenterophthalat ist, optional ein Polybutylenterephthalat.

26. Vorrichtung nach einem der vorangehenden Ansprüche, wobei die Zusammensetzung weiter ein Aromaöl umfasst.

27. Vorrichtung nach Anspruch 26, wobei das Aromaöl Pfefferminzöl ist.

28. Vorrichtung nach einem der vorangehenden Ansprüche, wobei die pharmazeutische Zusammensetzung eine Lösung des pharmazeutisch aktiven Agens in verflüssigtem HFC-134a oder HFC-227 enthält, mit dem Träger als ein Hilfslösungsmittol.

29. Vorrichtung nach einem der Ansprüche 1 bis 28, wobei die Zusammensetzung HFC-134a, Beclamethason-Dipropionat und Ethanol umfasst.

30. Verwendung eines Polyesters in Verbindung mit einer Zusammensetzung wie in einem der Ansprüche 1 bis 19 definiert.

31. Verwendung nach Anspruch 30. wobei der Polyester wie in Anspruch 24 oder 25 definiert ist.

## Revendications

1. Dispositif pour distribuer des doses pharmaceutiques, comprenant un récipient, rempli avec une composition pharmaceutique contenant un agent pharmaceutiquement actif dans une solution de 1,1,1,2-tétrafluoroéthane (HFC-134a) ou de 1,1,1,2,3,3,3-heptafluoropropane (HFC-227) liquéfié, et un support choisi parmi les alcools pharmaceutiquement acceptables, les polyols, les dérivés de (poly)alcoxy, les alkylesters d'acide gras, les polyalkylène glycols et le diméthylsulfoxyde, et des systèmes de valve disposés pour distribuer des doses aérosols de ladite composition pharmaceutique vers l'extérieur du récipient, dans lequel au moins une portion du dispositif est formée d'un polyester et la composition pharmaceutique ne comprend aucun trinitrate de glycéryle.

2. Dispositif selon la revendication 1, dans lequel le support est :
(a) un alcool alkyle inférieur en C₁-C₄ ou un alcool de lanoline ;
(b) un alcool polyalcoxy
(c) un éther ou ester polyoxyalkyle ; et/ou
(d) un alkyléther de polyoxyéthylène, un dérivé d'huile de castor polyoxyéthyléné, un ester d'acide gras de polyoxyéthylène sorbitane ou un stéarate de polyoxyéthylène.

3. Dispositif selon la revendication 2, dans lequel l'éther ou ester de polyoxyalkyle est un éther ou ester de polyoxyéthylène.

4. Dispositif selon la revendication 1, dans lequel le support est l'oléate d'éthyle, le myristate d'isopropyle, le palmitate d'isopropyle, le polyéthylène glycol, le propylène glycol, le glycérol, l'alcool isopropylique, l'éthanol et/ou le 2-(2-éthoxyéthoxy)éthanol (Transcutol).

5. Dispositif selon l'une quelconque des revendications précédentes, dans lequel la composition comprend jusqu'à 50% w/w de support et/ou entre 50 et 99% w/w de HFC-134a ou HFC-227.

6. Dispositif selon la revendication 5, dans lequel la composition comprend jusqu'à 25% w/w de support.

7. Dispositif selon la revendication 5 ou 6, dans lequel la composition comprend entre 75 et 95% w/w de HFC-134a ou HFC-227.

8. Dispositif selon l'une quelconque des revendications précédentes, dans lequel la composition comprend en outre un tensioactif organique.

9. Dispositif selon la revendication 8, dans lequel le tensioactif organique est l'alcool oléylique, le trioléate de sorbitane, le mono-oléate de sorbitane, le monolaurate de sorbitane, le monolaurate de polyoxyéthylène sorbitane (20), le mono-oléate de polyoxyéthylène sorbitane (20), la lécithine naturelle, l'éther oléylique de polyoxyéthylène (2), l'éther stéarylique de polyoxyéthylène (2), l'éther laurylique de polyoxyéthylène (4), les copolymères blocs d'oxyéthylène et d'oxypropylène, l'acide oléique, la lécithine synthétique, le dioléate de diéthylène glycol, l'oléate de tétrahydro-furfuryle, l'oléate d'éthyle, le myristate d'isopropyle, le mono-oléate de glycéryle, le monostéarate de glycéryle, le monoricinoléate de glycéryle, l'alcool cétylique, l'alcool stéarylique, le chlorure de pyridinium cétylique, l'huile d'olive, le monolaurate de glycéryle, l'huile de maïs, l'huile de graines de coton ou l'huile de graines de tournesol.

10. Dispositif selon la revendication 8, dans lequel le tensioactif est l'alcool oléylique.

11. Dispositif selon la revendication 1, dans lequel l'agent pharmaceutiquement actif est choisi parmi :
(1) les stéroïdes ;
(2) les agents antibiotiques et antibactériens ;
(3) les médicaments à action systémique ;
(4) les antihistaminiques ;
(5) les agents anti-inflammatoires ;
(6) les agents anticholinergiques ;
(7) les anti-émétiques ;
(8) les substances hormonales ;
(9) les bronchodilatateurs ;
(10) les médicaments sympathomimétiques ;
(11) les antifongiques ;
(12) les anesthésiants locaux ; et
(13) les sels pharmaceutiquement acceptables de l'un quelconque des précédents.

12. Dispositif selon la revendication 11, dans lequel :
(1) les stéroïdes sont la béclométhasone, la bétaméthasone, la dexaméthasone, la fluticasone, l'hydrocortisone, le budésonide, le flunisolide, la triamcinolone, la fluméthasone et la prednisolone ;
(2) les agents antibiotiques et antibactériens sont la néomycine, la mupirocine et la chlorhexidine ;
(3) les médicaments à action systémique sont le dinitrate d'isosorbide, le mononitrate d'isosorbide, l'apomorphine et la nicotine ;
(4) les antihistaminiques sont l'azélastine, la chlorphéniramine, l'astémizole et la terfénadine ;
(5) les agents anti-inflammatoires sont le piroxicam, le nédocromil, le cromoglycate, la fusafungine et l'iodoxamide ;
(6) les agents anticholinergiques sont le bromure d'ipratropium et le bromure d'oxitropium ;
(7) les anti-émétiques sont la dompéridone, l'hyoscine, la cinnarizine, le métoclopramide, la cyclizine, le dimenhydrinate et la prométhazine ;
(8) les substances hormonales sont la vasopressine et la desmopressine ;
(9) les bronchodilatateurs sont le salbutamol, le fénotérol et le salmétérol ;
(10) les médicaments sympathomimétiques sont la tramazoline et la xylométazoline ;
(11) les antifongiques sont le miconazole ; et
(12) les anesthésiants locaux sont la benzocaïne et la lignocaïne.

13. Dispositif selon la revendication 11 ou 12, dans lequel l'agent actif est le dipropionate de béclométhasone.

14. Dispositif selon la revendication 13, dans lequel le dipropionate de béclométhasone est dans un état anhydre, hydraté ou solvaté.

15. Dispositif selon la revendication 14, dans lequel le dipropionate de béclométhasone est dans un état anhydre.

16. Dispositif selon l'une quelconque des revendications précédentes, sensiblement exempt d'acide organique faible ou inorganique fort.

17. Dispositif selon l'une quelconque des revendications précédentes, dans lequel la solubilité de l'agent actif dans le HFC-134a ou HFC-227 liquéfié va de 3 à 0,001% w/v.

18. Dispositif selon la revendication 17, dans lequel la solubilité de l'agent actif dans le HFC-134a ou HFC-227 liquéfié va de 1 à 0,01% w/v.

19. Dispositif selon l'une quelconque des revendications précédentes, dans lequel le système de valves comprend au moins un composant formé en un polyester.

20. Dispositif selon la revendication 19, dans lequel deux composants de valve déplaçables relativement l'un par rapport à l'autre sont formés en un polyester.

21. Dispositif selon la revendication 20, dans lequel les deux composants de valve déplaçables relativement l'un par rapport à l'autre sont mutuellement coulissables.

22. Dispositif selon la revendication 19, dans lequel ledit composant est une chambre de dosage et/ou un coeur de valve.

23. Dispositif selon l'une quelconque des revendications précédentes, dans lequel le récipient comprend un corps de canette comprenant un polyester ou un corps de canette formé en un métal doublé de polyester.

24. Dispositif selon l'une quelconque des revendications précédentes, dans lequel le polyester est un dicarboxylate de polyalkylène benzène.

25. Dispositif selon la revendication 24, dans lequel le polyester est un polyalkylène téréphtalate, éventuellement un polybutylène téréphtalate.

26. Dispositif selon l'une quelconque des revendications précédentes, dans lequel la composition comprend en outre une essence aromatisante.

27. Dispositif selon la revendication 26, dans lequel l'essence aromatisante est l'huile essentielle de menthe.

28. Dispositif selon l'une quelconque des revendications précédentes, dans lequel la composition pharmaceutique comprend une solution de l'agent pharmaceutiquement actif dans du HFC-134a ou HFC-227 liquéfié, avec le support comme co-solvant.

29. Dispositif selon l'une quelconque des revendications 1 à 28, dans lequel la composition comprend du HFC-134a, du dipropionate de béclaméthasone et de l'éthanol.

30. Utilisation d'un polyester en contact avec une composition selon l'une quelconque des revendications 1 à 19.

31. Utilisation selon la revendication 30, dans laquelle le polyester est tel que défini dans la revendication 24 ou 25.
